# EUROPEAN PATENT SPECIFICATION

(11) **EP 2 547 669 B1**
(45) Date of publication and mention of the grant of the patent: **25.11.2015**
(21) Application number: 11716100.0
(22) Date of filing: 17.03.2011
(51) Int. Cl.: C07D 301/12, C07D 303/12, C08G 59/02, C08G 59/22, C08G 59/24

(54) **PROCESS FOR PREPARING DIVINYLARENE DIOXIDES**
VERFAHREN ZUR HERSTELLUNG VON DIVINYLARENDIOXIDEN
PROCÉDÉ DE PRÉPARATION DE DIOXYDES DE DIVINYLARÈNE

(30) Priority: 18.03.2010 US 315204 P
(43) Date of publication of application: 23.01.2013
(73) Proprietor: Blue Cube IP LLC, Midland MI 48674 (US)
(72) Inventor: GULYAS, Gyongyi, Lake Jackson TX 77566 (US); BHARADWAJ, Ashwin, R., Pearland TX 77584 (US); NULL, Marty, J., Lake Jackson TX 77566 (US)
(74) Representative: Beck Greener
(86) International application number: PCT/US2011/028800
(87) International publication number: WO 2011/116180

(56) References cited:
- WO-A1-95/05370
- US-A- 5 250 280
- US-A- 5 962 547
- WEIMING ZHU ET AL: "Oxidation of alkenes with aqueous potassium peroxymonosulfate and no organic solvent", THE JOURNAL OF ORGANIC CHEMISTRY, vol. 56, no. 25, 1 December 1991 (1991-12-01), pages 7022-7026, XP55000632, ISSN: 0022-3263, DOI: 10.1021/jo00025a014
- DENMARK S E ET AL: "CATALYTIC EPOXIDATION OF ALKENES WITH OXONE", JOURNAL OF ORGANIC CHEMISTRY, AMERICAN CHEMICAL SOCIETY, EASTON.; US, vol. 60, no. 5, 1 January 1995 (1995-01-01), pages 1391-1407, XP000917828, ISSN: 0022-3263, DOI: DOI:10.1021/JO00110A049

## Description

### BACKGROUND OF THE INVENTION

### Field of the Invention

The present invention is related to a process for preparing divinylarene dioxides, particularly divinylarene dioxides derived from divinylbenzene. More specifically, the present invention relates to a process for preparing a divinylarene dioxide by epoxidizing a divinylarene using a dioxirane.

### Description of Background and Related Art

Divinylarene dioxides, particularly divinylbenzene dioxide (DVBDO) and others which are derived from divinylbenzene (DVB) are a class of diepoxides which can be used as either a reactive diluent or as the main epoxy resin matrix in an epoxy thermoset formulation. DVBDO itself has a very low liquid viscosity (for example less than about 20 centipoise [0.02 Pas]) making DVBDO especially useful in the preparation of low viscosity epoxy formulations. The epoxy formulations made from DVBDO are useful as intermediates in the production of various other products. For example, epoxy formulations made from DVBDO are suitable for use in the fields of coatings, composites, and molding compositions.

Previously known processes for the preparation of DVBDO include for example using hydrogen peroxide (H₂O₂) as the oxidant in the reaction process. However, none of these previously known prior art processes can produce DVBDO in high yields efficiently and economically (for example, greater than 30 percent (%) yield). For example, the process described in Inoue et al, Bull. Chem. Soc. Jap., 1991, 64, 3442, employs a molybdenum catalyst and sodium nitrate or sodium sulfate additives providing yields of DVBDO at less than 10 % because of product instability and catalyst deactivation.

Worzakowska M, J. Appl. Polym. Sci., 2007, 103, 462 discloses that DVBDO is prepared using a magnesium oxide catalyst in acetonitrile. Equimolar amounts of acetamide, an undesirable side-product, are also co-produced in the Worzakowska process. No isolated yields of DVBDO are mentioned in the Worzakowska reference; only the ratio of mono- and di-epoxide derivatives of DVB, which is 5, is mentioned.

There are also known processes for the preparation of DVBDO using other reactants which do not include H₂O₂. For example, a process for preparing DVBDO using peracetic acid is described in U.S. Patent No. 2,982,752. The epoxidation process disclosed in U.S. Patent No. 2,982,752 yields an acetic acid side-product which can cause serious yield losses since divinyl arene oxides and dioxides show high acid sensitivity.

Hopff et al., Helvetica Chimica Acta, (1957), 40, 274; and German Patent No. DE 1079614 describes preparing DVBDO by reducing chloroacetylbenzenes with lithium aluminum hydride, and subsequently removing HCl from chlorohydrins in 32 % yield.

Sulfonium ylides for the preparation of epoxides from carbonyl compounds are described in Corey et al., J. Am. Chem. Soc., 1962, 84 (5), pp. 867-868. The reaction of sulfonium ylides and carbonyl compounds is successfully used for the synthesis of m-DVBDO from formaldehyde and m-(phenylenedimethylene)-dimethylsulfonium bromide in an alkaline medium providing 70 % yield. However, the practical application of the above process is hampered by the long four-day production of the sulfonium bromide as described in U.S. Patent No. 3,455,967.

U.S. Patent No. 5,962,547 discloses the preparation of DVBDO using Oxone® (trademark of E. I. du Pont de Nemours and Company) in an acetone-water reaction mixture with no catalyst. The process described in U.S. Patent No. 5,962,547 produces DVBDO in quantitative yield. Oxone contains 1 mol KHSO₄ and 1 mol K₂SO₄ besides the active component of 2 mol KHSO₅. In the process of U.S. Patent No. 5,962,547, the use of 2 mol excess of Oxone per mol of DVB is taught which means that four mol of KHSO₅ was used per mol of DVB, resulting in the generation of large amounts of sulfate side-products originating from the excess of oxidant and the accompanying sulfates in the oxidant.

The above processes known in the prior art all suffer from the disadvantage of generating residual products from the oxidizing agent. These undesired residual products, such as acids when peracids are the oxidants or sulfides when sulphonium salts are used, must be separated from the desired DVBDO product. An equivalent amount of acetamide is produced in the acetonitrile/H₂O₂ system. Salts are side-products formed when chlorohydrins are treated with a base or when Oxone is used. In addition, the different known preparations usually provide low yields of DVBDO. The process disclosed by Corey et al. above describes a 70 % yield of DVBDO; however, as aforementioned, that process is based on formaldehyde and a sulfonium salt; and such process requires an inconveniently long reaction time period, for example as much as four days.

The disadvantages of above processes make such processes unsuitable for industrial scale preparation of divinylarene dioxides. Accordingly, it is desired to develop a process for the successful preparation of divinylarene dioxides using an oxidant while minimizing the co-production of undesirable side-products; and provide an economical and efficient process wherein a divinylarene dioxide product is produced in high yields.

### SUMMARY OF THE INVENTION

The present invention advantageously provides a process for successfully preparing divinylarene dioxides at high yields (e.g. greater than about 60 %), at high selectivities for epoxide formation, and at a relative lower cost than previous known processes without the problems of the prior art processes such as co-production of undesirable acidic side-products or excessive amounts of salts.

One embodiment of the present invention is directed to a process for preparing a divinylarene dioxide including reacting (a) at least one divinylarene; (b) a partially neutralized sulfuromonoperoxoic acid as an oxidant; (c) at least one basic compound; (d) a ketone which reacts with the partially neutralized sulfuromonoperoxoic acid to form a dioxirane oxidant and which solubilizes the organic compounds present in the reaction mixture; wherein the ketone is selected from acetone; methyl-ethyl ketone; acetophenone; trifluoroacetone; trifluoro acetophenone; tetrafluoro acetophenone; or mixtures thereof; wherein the dioxirane oxidant is isolated and then the dioxirane oxidant is used in a subsequent process; or wherein the dioxirane is used *in situ* as the oxidant in the presence of the divinylarene mixed with the ketone during the addition of partially neutralized sulfuromonoperoxoic acid; under conditions to form a divinylarene dioxide product.

The present invention process for producing divinylarene dioxides uses an oxidant, such as partially neutralized sulfuromonoperoxoic, such as for example Caro's acid, suitable for obtaining high yields of a divinylarene dioxide using the process of the present invention and generating minimal amounts of salt wastes. The present invention process is particularly suited for the preparation of divinylbenzene dioxide (DVBDO), a very low viscosity liquid epoxy resin, from divinylbenzene (DVB).

Advantageously, the present invention process is carried out under conditions such that the co-production of undesirable side-products is minimized. In addition, the process of the present invention advantageously produces divinylarene dioxides in high yields, for example, in yields of greater than about 60 %.

### BRIEF DESCRIPTION OF THE DRAWINGS

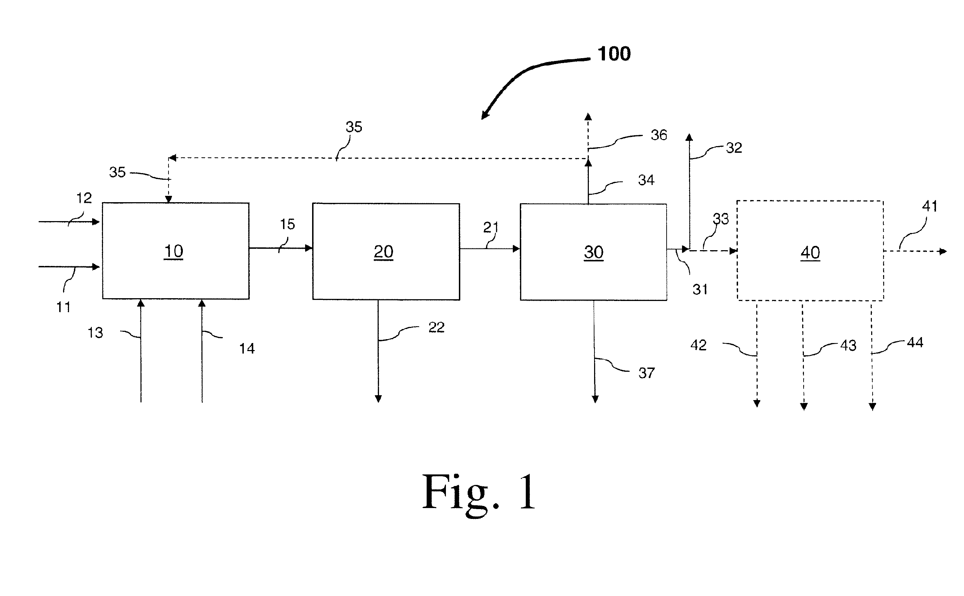

For the purpose of illustrating the present invention, the following drawings show a form of the present invention which is presently preferred. However, it should be understood that the present invention is not limited to the precise arrangements and pparatuses shown in the drawings. In the accompanying drawings, like reference numerals are used to denote like parts throughout the several drawings. Figure 1 is a block flow diagram showing one embodiment of the overall process of the present invention using an organic solvent extraction operation to separate a divinylarene dioxide product from a reaction effluent.

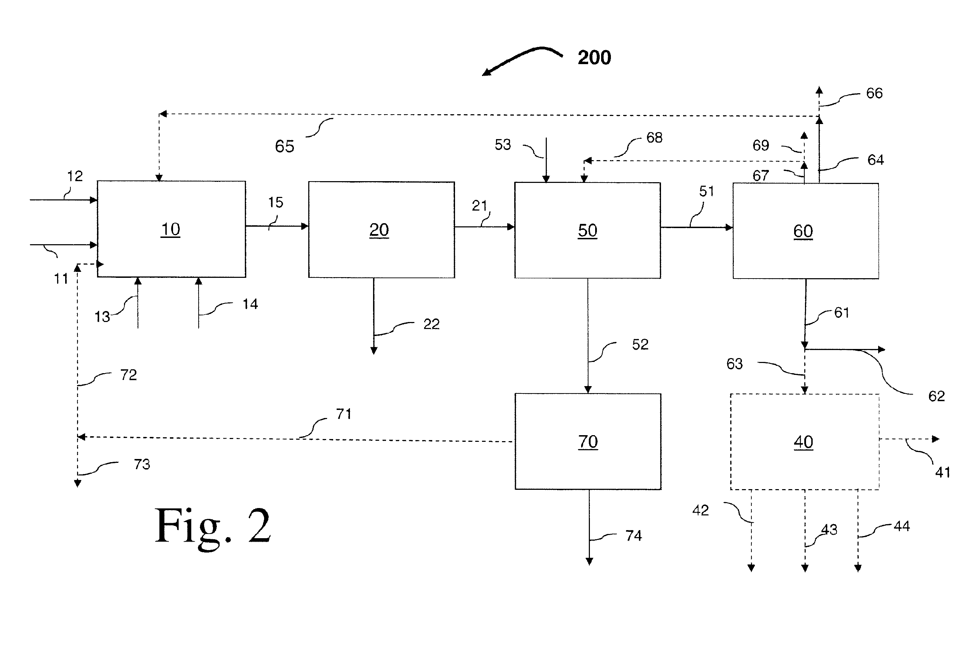

Figure 2 is a block flow diagram showing another embodiment of the overall process of the present invention using a phase separation operation to separate a divinylarene dioxide product from a reaction effluent.

### DETAILED DESCRIPTION OF THE INVENTION

The present invention is directed to an economically favorable process for preparing a divinylarene dioxide. A divinylarene dioxide of the present invention is prepared by reacting a divinylarene with a partially neutralized sulfuromonoperoxoic acid or Caro's acid in the presence of a basic compound and a ketone; wherein the reagents and molar ratios are selected to minimize the amounts of salt waste generated from the oxidant.

In another embodiment, a divinylarene dioxide such as DVBDO may be prepared by dissolving a divinylarene such as DVB in acetone, mixing the solution with a basic aqueous buffer, and using partially neutralized Carols acid as the oxidant. In this embodiment, acetone may also serve as a catalyst. Then the reaction may be carried out at a temperature of between about 0 °C to about 80 °C. After the epoxidation step is completed, the resulting product can be removed from the reaction mixture; and if desired, the resulting product may be purified by known means such as distillation.

In the economically favorable process of the present invention, the amount of side-products formed from the oxidizing agent, are minimized. In U.S. Patent Application Serial No. 61/315,200, filed of even date hereof, incorporated herein by reference, Oxone is described as an oxidant for the epoxidation of divinyl arenes. Oxone is also known as a "triple salt", since 1 mol Oxone is composed of 1 mol KHSO₄, 1 mol K₂SO₄ and 2 mol of KHSO₅, wherein the KHSO₅, a peroxomonosulfate, is the active component. Consequently, an aqueous waste that results from carrying out the process contains KHSO₄, K₂SO₄ that is present in Oxone and KHSO₄ that forms after KHSO₅ loses its oxygen in the epoxidation reaction.

U.S. Patent Application Serial No. 61/315,200, filed of even date hereof also describes the minimization of sulfate side-product formation in the process by the minimization of the excess of Oxone that is used for epoxidation.

One way to further minimize the sulfate side-product formation in the process of the present invention is to use a partially neutralized sulfuromonoperoxoic acid such as partially neutralized Caro's acid solution instead of the triple salt. For example, Caro's acid (H₂SO₅) may be generated from sulfuric acid or oleum and H₂O₂ followed by a partial neutralization such as for example by the addition of one equivalent base such as sodium hydroxide, potassium hydroxide, sodium carbonate, potassium carbonate, or mixtures thereof to the Caro's acid solution. WO 95/05370 discloses the epoxidation of alkenes such as cyclohexene and styrene using a partially neutralized Caro's acid solution. The process comprises contacting the alkene with a solution of partially neutralized Caro's acid in the presence of a ketone at a pH of from 7 to 8 under heterogeneous reaction conditions "Partially neutralized" herein means the addition of one equivalent base to Carols acid. This partially neutralized Caro's acid solution could be directly used in the epoxidation process of divinyl arenes with appropriate buffering minimizing, preferably eliminating, the presence of the inactive components KHSO₄ and K₂SO₄ from the oxidant decreases the salt waste generated by 50 %. To further improve process economics the sulfate salt by-product can be isolated, purified to the appropriate levels and used in different industrial and agricultural applications. Examples of industrial fields that use potassium sulfate include accelerator in the manufacture of wallboards; reagent component in paper pulping; flash suppressant for military applications; potassium supplement in food; component in aqueous based drilling fluids and analytical reagent. The most important agricultural application is the use of potassium sulfate as a fertilizer; and to lesser extent in animal feed.

Oxone is generally made from Caro's acid by an alkaline treatment with a potash solution, a potassium carbonate and potassium hydroxide based system, to form the triple salt. The process to make this "triple salt" is described, for example, in U.S. Patent No. 5, 607, 656 (herein "the '656 patent"), incorporated herein by reference. The process of the '656 patent involves the oxidation of sulfuric acid or oleum with H₂O₂, to generate the active component, Caro's acid. Then, the resulting highly acidic mixture is neutralized with potash to obtain a solution of the triple salt. The triple salt then has to be crystallized which involves the evaporation of water to concentrate the aqueous solution. The solids then need to be filtered, washed and dried. Consequently, the energy requirement of the process described in the '656 patent is quite high.

Although improvements to the process of the '656 patent have been made such as removing the cold crystallization steps to isolate solid Oxone, the process of the '656 patent is still energy inefficient after the water evaporation process. The solid must be transferred dried and the process apparatus must be further cleaned before any recycling or analysis of the filtrate is completed to obtain more solid Oxone. Isolation of the solid may also require additional equipment for drying the triple salt.

In the present invention, a partially neutralized Caro's acid, used as an active oxidant, heretofore has not been described or used in the art. The acidic solution is neutralized with the appropriate base such as a potash solution and the resulting solution is used as the oxidant. The simplified process of the present invention eliminates the formation of sulfate waste that would be generated from the sulfate salts originating from the addition of the extra sulfuric acid necessary for the preparation and crystallization of the triple salt. Also significantly decreases the energy needs of the process, since no water evaporation, crystallization, filtration and drying is involved. Furthermore, the oxidant used in the present invention is preferably in an aqueous solution. Partial neutralization of the Caro's acid, results in a solution of the oxidant and can directly be used for the epoxidation. In contrast, when Oxone, the solid triple salt is used, the Oxone needs to be solubilized which requires additional amounts of water.

In its broadest scope, the present invention comprises a process for preparing a divinylarene dioxide including reacting (a) at least one divinylarene; (b) an oxidant, wherein the oxidant is partially neutralized Caro's acid; (c) at least one basic compound, and (d) a ketone which reacts with the partially neutralized sulfuromonoperoxoic acid to form a dioxirane oxidant and which solubilizes the organic compounds present in the reaction mixture under conditions to form a divinylarene dioxide. Optionally, the reaction mixture of the present invention process may include other desirable additives.

The divinylarene and the other components above, are contacted with the neutralized Caro's acid oxidizing agent in a reactor, which may be batch or continuous; and the reactants are allowed to react to produce the corresponding divinylarene dioxide. The co-produced salts, the other components left in the reactor, may be separated from the product to give a usable divinylarene dioxide product. The divinylarene dioxide product may optionally be purified, for example, by distillation, crystallization, or other purification methods known in the art. The co-produced salts maybe purified and used in other industrial or agricultural applications.

One of the examples of the present invention is directed to a process for preparation of DVBDO by epoxidation of DVB with dioxiranes, wherein the dioxiranes can be isolated or generated in-situ as described in R.W. Murray, J. Org. Chem., 50 (16) 2847, 1985, incorporated herein by reference. The in-situ generation of dioxiranes requires a ketone and a strong oxidizing agent. The strong oxidizing agent of the present invention is partially neutralized Caro's acid. The ketone component acts as a catalyst and converts back to its original state after the oxidation reaction.

In one embodiment, DVB in an appropriate solvent such as acetone and in the presence of an aqueous solution of the basic compound such as an inorganic salt or base are treated with the oxidizing agent; and after the proper incubation time DVBDO is obtained as the major reaction product with complete DVB conversion; with no or a very minor amount (less than about 5 %) of the monooxide of divinylbenzene (DVBMO) being formed. Then the reaction product can be extracted into an organic solvent, water washed, optionally filtered, then distilled to give a highly pure DVBDO product. The co-products are sulfates that can stay in the aqueous phase, or precipitate as solids, providing a very simple way of separation. The ketone component can be recycled and reused. It can be isolated from the reaction mixture by precipitation or extraction or distillation.

In another embodiment, DVB in an appropriate solvent such as acetone and in the presence of an aqueous solution of the basic compound such as an inorganic salt or base are treated with the proper amount of the oxidizing agent; and after the proper incubation time DVBDO is obtained as the major reaction product with complete DVB conversion; with minor amount less than about 40 % of the monooxide of divinylbenzene (DVBMO) being formed. Then the reaction product can be extracted into an organic solvent, water washed, optionally filtered, then the mono and di-epoxides are separated with appropriate methods such as distillation.

As an illustration of one embodiment of the present invention, for example, a divinylarene dioxide such as DVBDO is prepared by dissolving a divinylarene such as DVB in acetone. Acetone can also serve as the ketone catalyst and the solvent. The solution is mixed with an aqueous solution of the basic compound such as sodium hydrogen carbonate. Then the oxidizing agent for example partially neutralized Caro's acid may be added to the reaction mixture; and then the reaction may be carried out at a temperature of between about 0 °C to about 80 °C. After the epoxidation is completed, the solvent, salts, and other materials may be removed from the product, and if desired, the product may be purified by known means such as distillation. The solvent can be recycled and reused. And the co-produced sulfate salts can be purified and used in other industrial and agricultural applications,

The source of divinylarene useful in the present invention may come from any known sources and particular to known processes for the preparation of divinylarenes.

In one embodiment of the present invention, the divinylarene useful in the present invention may comprise any substituted or unsubstituted arene nucleus bearing two vinyl groups in any ring position. The arene may include for example benzene, substituted benzenes, or (substituted) ring-annulated benzenes, and mixtures thereof. In one embodiment, divinylbenzene may be *ortho, meta*, or *para* isomers or any mixture thereof. Additional substituents may consist of oxidation-resistant groups including for example saturated alkyl, aryl, halogen, nitro, isocyanate, or RO- (where R may be saturated alkyl or aryl), or mixtures thereof. Ring-annulated benzenes may include for example naphthlalene, tetrahydronaphthalene, and the like, and mixtures thereof.

In another embodiment, the divinylarene may contain quantities of substituted arenes. The amount and structure of the substituted arenes depend on the process used in the preparation of the divinylarene. For example, DVB prepared by the dehydrogenation of diethylbenzene (DEB) may contain quantities of ethylvinylbenzene (EVB) and DEB. The amount of EVB is preferably less than 50 %.

The divinylarene used in the process of the present invention may include for example divinylbenzene, divinylnaphthalene, divinylbiphenyl, divinyldiphenylether; and mixtures thereof.

The concentration of the divinylarene used in the present invention may range generally from about 0.1 weight percent (wt %) to about 70 wt %, preferably from about 1 wt % to about 65 wt %, more preferably from about 2 wt % to about 60 wt %, and most preferably from about 4 wt % to about 50 wt %.

The sulfuromonoxoperoxoic acids useful in the present invention as component (b) generally contain sulfur, oxygen and hydrogen atoms. Caro's acid is one example of a sulfuromonoxoperoxoic acid. Caro's acid, H₂SO₅, is also known as peroxymonosulfuric acid. Caro's acid is a strong oxidizing compound. Caro's acid may be produced by reacting a sulfuric acid such as concentrated sulfuric acid, or oleum with hydrogen peroxide to produce Caro's acid. For example, a process for preparing Caro's acid is described in U.S. Patent No. 5,139,763 incorporated herein by reference.

The oxidizing agent or oxidant, component (b), useful in the present invention may comprise one or more of the following: (1) a partially neutralized Caro's acid in addition to a ketone which generates an oxidant *in situ;* (2) partially neutralized Caro's acid in addition to a ketone which generates an dioxirane oxidant which is isolated; or (3) mixtures thereof.

For example, a partially neutralized Caro's acid may be obtained by neutralizing Caro's acid with one or more of the following: (i) an inorganic base, (ii) an ammonium compound, (iii) an alkylammonium base; (iv) an arylammonium base; (v) a mixed alkyl-arylammonium base; (vi) an alkylphosphonium base; (vii) an arylphosphonium base; (viii) mixed alkyl-aryl phosphonium base; or (ix) mixtures thereof.

In the present invention, a partially neutralized Caro's acid may be mixed with a ketone which results in the formation of a dioxirane oxidant *in situ.* The dioxirane may be isolated and then used in a subsequent process; or the dioxirane may be generated from Caro's acid and the ketone in the presence of the divinylarene and used *in-situ* as the oxidant of the present invention.

The molar ratio of the oxidant used in the present invention generally includes from about 0.5 to about 10 equivalents per double bond, preferably from about 1 equivalent to about 4 equivalents, and more preferably from about 1.1 equivalents to 2.5 equivalents.

The basic compound, component (c), useful in the present invention includes inorganic additives such as bases or salts that are capable of buffering the aqueous phase and providing an optimal pH for the reaction. For example, the inorganic additive can be sodium or potassium carbonate; hydrogen carbonate; sodium or potassium hydroxide; dibasic sodium or potassium phosphate; and mixtures thereof.

The concentration of the basic compound used in the present invention may range generally from about 0.05 wt % to about 30 wt %, preferably from about 0.1 wt % to about 20 wt %, and more preferably from about 1 wt % to about 10 wt % based on the aqueous phase of the composition.

The ketone compound useful in the present invention can be for example chiral or achiral and may be for example, acetone, methyl- ethyl ketone, fluorinated ketones (trifluoroacetone, tri or tetrafluoro acetophenone), chiral ketones, and mixtures thereof.

The ratio of the ketone in the present invention may range from about 0 to about 500 mol% (referenced to DVB), preferably from about 1 mol% to about 200 mol%, and more preferably from about 10 mol% to about 100 mol%.

The ketone solvent useful in the process of the present invention also serves as the catalyst. The concentration of the solvent used in the present invention may range generally from 0 wt % to about 90 wt %, preferably from about 25 wt % to about 75 wt %, and more preferably from about 35 wt % to about 60 wt %, based on the total weight of the composition.

Another optional component useful in the present invention may include for example a phase transfer agent. The phase transfer agent useful in the present invention may be for example, tetraalkyl, tetraphenyl, or mixed alkyl and aryl ammonium or phosphonium salts; and mixtures thereof. Preferably, the phase transfer salts may include for example Bu₄NHSO₄, Bu₄NCl and mixtures thereof.

The ratio of the phase transfer agent in the present invention may range from 0 mol% to about 25 mol% (referenced to DVB), preferably may range from about 1 mol% to about 20 mol %, and more preferably from about 2 mol% to about 10 mol%.

An example of another optional component useful in the present invention may include chelating agents to remove trace metal impurities to stabilize the oxidizing agent. The chelating agent useful in the present invention may be for example phosphates such as K₂HPO₄ or ethylenediamine tetraacetic acid (EDTA), diethylenetriamine pentaacetic acid (DTPA), and similar chelants with phosphonate groups such as ethylenediaminetetramethylene-tetraphosphonic acid (EDTMP) and the like. A generalized structure for the chelants is depicted as follows: 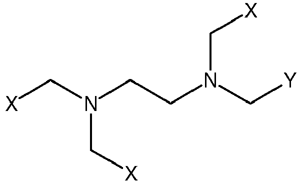 wherein X could be carboxylic acid or phosphonic acid groups or their alkali metal salts and Y could be equal to X or -CH₂-N(CH₂-X)₂.

The concentration of the chelating agent used in the present invention may range generally from 0 wt % to about 20 wt %, preferably from about 0.01 wt % to about 10 wt %, and more preferably from about 0.05 wt % to about 5 wt %, based on the total weight of the composition.

Numerous additives can optionally be employed as part of the present invention including for example, a free radical polymerization inhibitor. For example, one or more free radical polymerization inhibitors may be added to any of the steps of the process of the present invention including for instance the reaction step, the recovery step and/or the purification step. The inhibitor may comprise a phenol; a hydroquinone; a quinone; an aromatic nitro compound, a nitrophenol, an amine; a nitroso compound; a nitroxide; or mixtures thereof.

Free radical polymerization inhibitors which may be employed in the present invention, include for example phenols such as 4-methoxy phenol, 4-tert-butylcatechol, or 2,6-di-tert-butyl-4-methylphenol; hydroquinones such as 1,4-dihyrdroxybenzene or 3,5-di-tert-butylbenzene-1,2-diol; quinones such as 1,4-benzoquinone or naphthalene-1,2-dione; aromatic nitro compounds such as 1,3-dinitrobenzene or 1,4-dinitrobenzene; nitrophenols such as 2-(sec-butyl)-4,6-dinitrophenol, 4-methyl-2-nitrophenol, or 4-methyl-2,6-dinitrophenol; amines such as phenothiazine, N¹-phenyl-N⁴-propylbenzene-1,4-diamine, N-(1,4-dimethylpentyl)-N'phenyl-p-phenylenediamine, N,N-diethylhydroxylamine; nitroso compounds such as N-nitrosophenylhydroxylamine ammonium salt; nitroxide compounds such as bis(1-oxyl-2,2,6,6-tetramethylpiperidin-4-yl) sebacate, 1-oxyl-2,2,6,6-tetramethylpiperidine, 1-oxyl-2,2,6,6-tetramethylpiperidin-4-ol, 1-oxyl-2,2,6,6-tetramethyl-4-n-butoxypiperidine; or mixtures thereof.

The concentration of the inhibitors used in the present invention can be for example from about 0.01 wt % up to about 5 wt % based on the divinylarene added. Preferably it should range from about 0.1 wt % up to about 2 wt % based on the divinylarene added.

An assortment of optional additives may be added to the composition of the present invention including for example, other resins, stabilizers, fillers, plasticizers, catalyst de-activators, and the like; and mixtures thereof.

The concentration of the optional additives used in the present invention may range generally from 0 wt % to about 99.9 wt %, preferably from about 0.1 wt % to about 99.9 wt %, more preferably from about 1 wt % to about 99 wt %, and most preferably from about 2 wt % to about 98 wt %.

The preparation of divinylarene dioxides with the minimization of the co-production of undesirable side-products may be achieved by (i) adding to a reactor the following reactants: at least one divinylarene; the basic compound or its aqueous solution; optionally, and a ketone; (ii) contacting the reactants with partially neutralized Caro's acid; and then (iii) allowing the reactant components to react under reaction conditions to produce the corresponding divinylarene dioxide. In the process, the basic compound may be added at the beginning of the reaction, simultaneously or intermittently with the oxidant.

The reaction conditions to manufacture the divinylarene dioxides, such as DVBDO, include carrying out the reaction of the reactants under a temperature, generally in the range of from about 0 °C to about 80 °C, preferably from about 5°C to about 60°C, and more preferably from about 20 °C to about 50 °C. The pressure of the reaction may be generally from about 10.1 k Pa to about 1013 k Pa. The pH of the reaction may be controlled to a pH of from about 5 to about 12, preferably from about 6 to about 10, and more preferably from about 7 to about 8.

The reaction process of the present invention may be a batch or a continuous process. The reactor used in the process may be any reactor and ancillary equipment well known to those skilled in the art.

In addition the reaction above, the process of the present invention may include further processing steps such as a method of separating any co-products formed during the reaction from product. The separation method may include any separation process and equipment well known to those skilled in the art.

During the reaction for the preparation of divinylarene dioxides, equivalent amount of potassium sulfate side-product forms that can be removed by (i) filtration and with the separation of organic and aqueous components of the reaction mixture; or (ii) by extraction of the divinylarene dioxide product with a non-miscible organic solvent, followed by the appropriate water washes of the organic phase. Other undesirable oxidized by-products and derivatives, such as for example carbonyl compounds and hydrolyzed epoxy products, are also not formed in any appreciable quantities using the process of the present invention.

After the reaction of the present invention, the undesirable by-products; and any remaining, catalyst, and solvent, may be removed to recover a usable divinylarene dioxide product. Then the product may optionally be purified by well-known means in the art such as by distillation, crystallization, precipitation, extraction and the like.

The solvent and catalyst components of the reaction mixture are separated from the divinylarene dioxide product by well-known means in the art such as by distillation, extraction and the like and recycled.

The salt by-product is separated from the reaction mixture by well-known means in the art such as by filtration. Residual organic content maybe removed by organic solvent washes, calcination, recrystallization, partial recrystallization or adsorption on activated carbon and the like.

In one embodiment of the process of the present invention, the process for preparing a divinylarene dioxide generally may comprise the steps of:
(A) contacting at least one divinylarene with partially neutralized Caro's acid and a basic compound or an aqueous solution of the basic compound; and a ketone; to produce a divinylarene dioxide product in a reaction mixture;
(B) separating the divinylarene dioxide product formed in step (a) from the reaction mixture of step (a);
(C) optionally, purifying the divinylarene dioxide product obtained in step (B);
(D) optionally, recovering and/or recycling the ketone from the reaction mixture of step (a); and
(E) optionally, recovering a sulfate by-product formed in step (A) from the reaction mixture of step (A) and purifying the sulfate by-product recovered from step (A).

The process for preparing a divinylarene dioxide of the present invention includes either(i) generating, *in-situ,* dioxirane from a ketone, and a partially neutralized Caro's acid; in a pH controlled reaction in the presence of a divinylarene. The pH can be controlled by introducing a buffer or a basic compound into the reactor and then delivering the oxidizing agent; or by the simultaneous delivery of the oxidizing agent and buffer or base; or(ii) reacting an isolated dioxirane generated from a ketone and partially neutralized Caro's acid with a divinylarene such as DVB in a pH controlled environment. The process may include one or more of the following optional steps: (iii) separating sulfate co-products from a divinylarene dioxide product such as DVBDO product, by filtration and/or extraction of DVBDO with an organic solvent; (iv) removing the ketone from a divinylarene dioxide product such as DVBDO product; (v) distilling a divinylarene dioxide product such as DVBDO product to give a high purity DVBDO product; (vi) recycling the ketone by separating the ketone from a divinylarene dioxide product/solvent by precipitation, distillation or extraction; (vii) regenerating Caro's acid, or (viii) isolating and purifying the sulfate by-product by organic solvent washes; calcination; recrystallization or adsorption of the organic contaminants onto activated carbon.

One advantage of the present invention process is that high yields of divinylarene dioxides may be produced by the process of the present invention. With high yields of divinylarene dioxides produced, the process of the present invention advantageously requires no recycle of divinylarene or divinylarene monooxide.

The "high yield" of the divinylarene dioxides produced by the process of the present invention, is generally greater than about 60 %; preferably greater than 75 % and more preferably greater than 85 %. In another embodiment, the yield is from about 60 % to about 100 %; preferably, ranges from about 70 % to about 100 %; more preferably, from about 80 % to about 100 %; and most preferably, from about 90 % to about 100 % based on divinylarene starting material.

Advantageously, by using a partially neutralized Caro's acid of the present invention, a lower generation of side-product sulfates can be achieved.

As an illustration of another embodiment of the process of the present invention, the process for preparing a divinylarene dioxide may include one or more the following optional steps: (ii) using appropriate oxidizing agent and divinylarene molar ratios that results in the formation of a divinylarene dioxide as the main product accompanied by a divinylarene monooxide as the minor component; (iv) separating sulfate co-products from a divinylarene oxidation products such as DVBDO and DVBMO products, by filtration and/or extraction with an organic solvent; (v) removing the ketone from a divinylarene oxidation product such as DVBDO and DVBMO product; (vi) distilling a divinylarene mono and dioxide product mixture such as DVBDO and DVBMO products to give a high purity major DVBDO and minor DVBMO product; (vii) recycling the ketone by separating the ketone from a divinylarene dioxide product/solvent by precipitation, distillation or extraction; (viii) regenerating Caro's acid; (ix) isolating the sulfate by-product by filtration; (x) purifying the sulfate by-product by organic solvent washes or calcination or crystallization or adsorption of the impurities onto activated carbon.

With reference to Figure 1, there is shown one embodiment of the process of the present invention generally indicated by numeral 100. The process of Figure 1 includes a reactor 10, a separation/filtration apparatus 20, an evaporation apparatus 30 and a purification/distillation apparatus 40. In Figure 1, there is shown a feed stream of divinylarene 11, a feed stream of ketone 12, a feed stream of a basic compound 13, and a feed stream of oxidant 14; all of the streams 11-14 which are fed into the reaction apparatus, reactor 10, for carrying out the epoxidation reaction step of the present invention. A recycle stream 34, 35 may also be introduced into reactor 10 (described below).

A product stream 15 exits from reactor 10 and may be introduced as a feed stream 15 to a separation/filtration apparatus 20, wherein solid and liquid components are separated. A liquid component stream 21 from apparatus 20 contains the divinylarene dioxide product, and stream 21 is sent for further processing to apparatus 30. A solid reaction component stream 22 (which may include a salt byproduct) is separated from the product liquid stream 21 and stream 22 exits apparatus 20.

The divinylarene dioxide product stream, stream 21 can be sent to the solvent removal/evaporator apparatus 30, for separation of the product from solvent/catalyst. The product stream from apparatus 30, stream 31, may be used without further purification as stream 31, 32; or optionally, as shown in dotted lines, the product stream 31 may be introduced to a purification/distillation apparatus 40 as feed stream 31, 33. An organic stream containing the ketone from apparatus 30, stream 34, may be removed from apparatus 30 and optionally recycled to reactor 10 as stream 34, 35. Optionally, stream 34 may be purged as stream 34, 36 or sent to an organic waste recovery unit not shown. An aqueous waste stream from reactor 30, stream 37 can be sent to a waste water treatment unit (not shown)

As aforementioned, the divinylarene dioxide product stream from apparatus 30, stream 31, 33 may optionally be introduced to a purification/distillation apparatus 40, to form a purified product stream, stream 41. Also if divinylarene monooxide accompanies the divinylarene dioxide product, the divinylarene monooxide can be separated as a byproduct stream 42 in the purification/distillation apparatus 40. The divinylarene monooxide byproduct leaves apparatus 40 as stream 42 and distillation bottoms as stream 43. Possible contaminants with lower boiling point than DVBDO, for example alkylvinylbenzene oxides or low levels of oxidation by-products can also be separated and removed from apparatus 40 as stream 44.

Any of the recycle streams of Figure 1 may require a periodic or continuous purge to limit the buildup of impurities.

With reference to Figure 2, there is shown another embodiment of the process of the present invention generally indicated by numeral 200. The process of Figure 2 includes a reactor 10 similar to that shown in Figure 1, a separation/filtration apparatus 20 similar to that shown in Figure 1, an extraction apparatus 50, a separation apparatus 60, a purification/distillation apparatus 40 similar to that shown in Figure 1, and a separation apparatus 70. In Figure 2, there is shown a feed stream of divinylarene 11, a feed stream of ketone 12, a feed stream of a basic compound 13, and a feed stream of oxidant 14 . All of which are fed into the reaction apparatus, reactor 10, for carrying out the epoxidation reaction step of the present invention. A recycle stream 71, 72 also is introduced into reactor 10 (described below).

The product stream 15 from reactor 10 may be introduced as feed stream 15 to a separation/filtration apparatus 20, wherein in the divinylarene dioxide product stream 21 also containing ketone, is separated from the salt by-product stream 22. In the embodiment shown in Figure 2, the divinylarene dioxide product stream, stream 21, is sent to an extraction apparatus, apparatus 50

Feed stream 21 is introduced into extraction apparatus 50, for separation from the aqueous and aqueous soluble components by extraction with an organic extraction solvent, stream 53 and a recycled extraction solvent, stream 67, 68. The extracted product in the extraction solvent is separated from the aqueous phase and may be introduced to the separation apparatus 60 as stream 51. An aqueous organic waste stream from apparatus 50, stream 52, may also be removed and introduced to a separator apparatus 70.

Stream 51, maybe introduced to separation apparatus 60, to separate from the extraction solvent. The purified divinylarene product stream, stream 61 leaving apparatus 60, can be used as is without further processing, as stream 61,62; or optionally, as shown in dotted lines, the stream 61 may be used for applications requiring high purity product and thus may be purified further and fed as stream 61,63 in a purification/distillation apparatus, apparatus 40. The extraction solvent, stream 67, 68 can be recycled into apparatus 50. Optionally, stream 67 may be purged as stream 67, 69 or sent to an organic waste recovery unit not shown. In another embodiment, the extraction solvent might dissolve some of the ketone which also can be separated from the extraction solvent in apparatus 60 and recycled to reactor 10 as stream 64, 65. Optionally, stream 64 may be purged as stream 64, 66 or sent to an organic waste recovery unit not shown. Product stream 61, 63 can be further purified in a purification/distillation apparatus, such as apparatus 40. Also if divinylarene monooxide accompanies the divinylarene dioxide product, the monooxide can also be separated in purification/distillation apparatus 40. High purity divinylarene product leaves apparatus 40 as stream 41. Divinylarene monooxide byproduct as stream 42 and distillation bottoms as stream 43. Possible contaminants with lower boiling point than DVBDO, for example alkylvinylbenzene oxides or low levels of oxidation by-products can also be separated and removed from apparatus 40 as stream 44.

Stream 52, the aqueous phase from apparatus 50, might contain catalyst/solvent. Stream 52 can be fed into a separation apparatus such as apparatus 70, to separate the water from the solvent/catalyst component. From apparatus 70, the solvent/catalyst component can be recycled as stream 71, 72 back to the epoxidation reactor 10. Optionally, stream 71 may be purged as stream 71, 73 or or sent to an organic waste recovery unit not shown. An aqueous waste stream from reactor 70, stream 74, can be sent to a waste water treatment unit (not shown)

Any of the recycle streams of Figure 2 may require a periodic or continuous purge to limit the buildup of impurities.

The divinylarene dioxides prepared by the process of the present invention, particularly those derived from divinylbenzene such as for example divinylbenzene dioxide (DVBDO), are class of diepoxides which have a relatively low liquid viscosity but a higher rigidity than conventional epoxy resins.

The divinylarene dioxide prepared by the process of the present invention may comprise, for example, any substituted or unsubstituted arene nucleus bearing two vinyl groups in any ring position. The arene portion of the divinylarene dioxide may comprise benzene, substituted benzenes, ring-annulated benzenes, substituted ring-annulated benzenes, or mixtures thereof. The divinylarene portion of the divinylarene dioxide may be *ortho, meta*, or *para* isomers or any mixture thereof. Additional substituents may consist of H₂O₂-resistant groups including saturated alkyl, aryl, halogen, nitro, isocyanate, or RO- (where R may be a saturated alkyl or aryl). Ring-annulated benzenes may comprise for example naphthlalene, tetrahydronaphthalene, and the like. Homologously bonded (substituted) benzenes may comprise for example biphenyl, diphenylether, and the like.

The divinylarene oxide product prepared by the process of the present invention may be illustrated generally by general chemical Structures I -IV as follows:

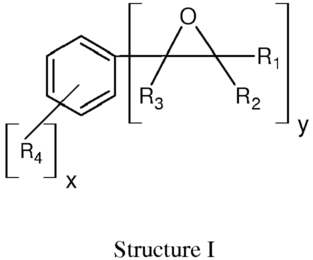

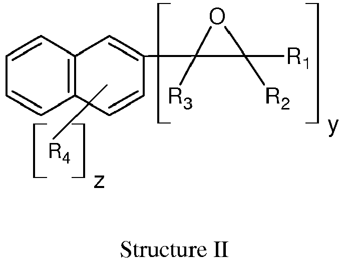

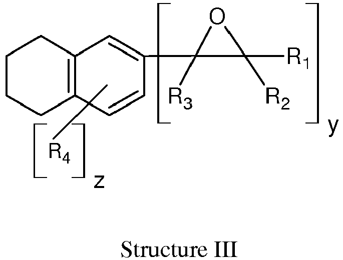

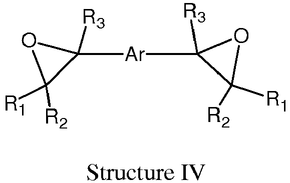

In the above Structures I, II, III and IV of the divinylarene dioxide product of the present invention, each R₁, R₂, R₃ and R₄ individually may be hydrogen, an alkyl, cycloalkyl, an aryl or an aralkyl group; or a oxidant-resistant group including for example a halogen, a nitro, an isocyanate, or an RO group, wherein R may be an alkyl, aryl or aralkyl; x may be an integer of 0 to 4; y may be an integer greater than or equal to 2; x+y may be an integer less than or equal to 6; z may be an integer of 0 to 6; and z+y may be an integer less than or equal to 8; and Ar is an arene fragment including for example, 1,3-phenylene group.

The divinylarene dioxide product produced by the process of the present invention may include for example alkyl-vinyl-arene monooxides depending on the presence of alkylvinylarene in the starting material.

In one embodiment of the present invention, the divinylarene dioxide produced by the process of the present invention may include for example divinylbenzene dioxide, divinylnaphthalene dioxide, divinylbiphenyl dioxide, divinyldiphenylether dioxide, and mixtures thereof.

In a preferred embodiment of the present invention, the divinylarene dioxide used in the epoxy resin formulation may be for example divinylbenzene dioxide (DVBDO). Most preferably, the divinylarene dioxide component that is useful in the present invention includes, for example, a divinylbenzene dioxide as illustrated by the following chemical formula of Structure V:

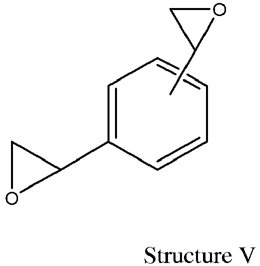

The chemical formula of the above DVBDO compound may be as follows: C₁₀H₁₀O₂; the molecular weight of the DVBDO is about 162.2; and the elemental analysis of the DVBDO is about: C, 74.06; H, 6.21; and O, 19.73 with an epoxide equivalent weight of about 81 g/epoxide equivalent.

Divinylarene dioxides, particularly those derived from divinylbenzene such as for example divinylbenzene dioxide (DVBDO), are class of diepoxides which have a relatively low liquid viscosity but a higher rigidity and crosslink density than conventional epoxy resins.

Structure VI below illustrates an embodiment of a preferred chemical structure of a divinylbenzene dioxide (DVBDO) useful in the present invention:

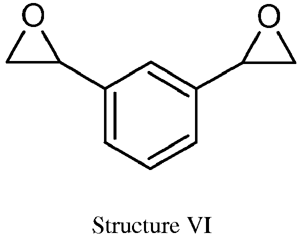

Structure VII below illustrates another embodiment of a preferred chemical structure of the DVBDO useful in the present invention:

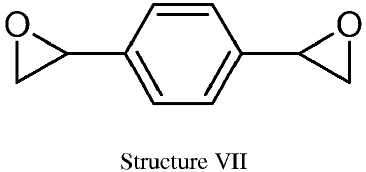

When DVBDO is prepared by the process of the present invention, it is possible to obtain one of three possible isomers: *ortho, meta*, and *para.* Accordingly, the present invention includes a DVBDO illustrated by any one of the above Structures individually or as a mixture thereof. Structures VI and VII above show the *meta* (1,3-DVBDO) and *para* isomers of DVBDO, respectively. The *ortho* isomer is rare; and usually DVBDO is mostly produced generally in a range of from about 9:1 to about 1:9 ratio of *meta* (Structure VI) to *para* (Structure VII) isomers. The present invention preferably includes as one embodiment a range of from about 6:1 to about 1:6 ratio of Structure VI to Structure VII, and in other embodiments the ratio of Structure VI to Structure VII may be from about 4:1 to about 1:4 or from about 2:1 to about 1:2.

In one embodiment, the process of the present invention is particularly suited for the preparation of divinylbenzene dioxide, a low viscosity liquid epoxy resin. The viscosity of the divinylarene dioxides produced by the process of the present invention ranges generally from about 10 mPa-s to about 100 mPa-s; preferably, from about 10 mPa-s to about 50 mPa-s; and more preferably, from about 10 mPa-s to about 25 mPa-s at 25 °C.

The utility of the divinylarene dioxides of the present invention requires their thermal stability to allow their formulation or processing at moderate temperatures (for example, at from about 100 °C to about 200 °C) for up to several hours (for example, for at least 2 hours) without oligomerization or homopolymerization. Oligomerization or homopolymerization during formulation or processing is evident by a substantial increase in viscosity or gelling (crosslinking). The divinylarene dioxides of the present invention have sufficient thermal stability such that they do not experience a substantial increase in viscosity or gelling during formulation or processing at moderate temperatures.

The divinylarene dioxide products of the present invention are useful for the preparation of epoxy resin compositions or formulations which, in turn, are useful for preparing thermosets or cured products in the form of coatings, films, adhesives, laminates, composites, electronics, and the like.

As an illustration of the present invention, in general, resin compositions based on the divinylarene dioxide products of the present invention may be useful for casting, potting, encapsulation, molding, and tooling. The present invention is particularly suitable for all types of electrical casting, potting, and encapsulation applications; for molding and plastic tooling; and for the fabrication of vinyl ester resin based composites parts, particularly for producing large vinyl ester resin-based parts produced by casting, potting and encapsulation. The resulting composite material may be useful in some applications, such as electrical casting applications or electronic encapsulations, castings, moldings, potting, encapsulations, injection, resin transfer moldings, composites, coatings and the like.

### EXAMPLES

The following examples and comparative examples further illustrate the present invention in detail but are not to be construed to limit the scope thereof. All chemicals used in the Examples were purchased form Aldrich and used without further purification. The partially neutralized Caro's acid used in the Examples was either (i) prepared in accordance with slightly modified literature procedures; or (ii) obtained from DuPont under the trade name ActXone™; as indicated in the Examples. The concentration of the partially neutralized Caro's acid solution was determined using standard iodometric titration as described in ASTM method E298-08.

The epoxidation product mixtures prepared in the Examples which follow were analyzed by standard gas chromatography (GC) and gel permeation chromatography analytical equipment and methods.

Total organic carbon (TOC) was measured using a Sievers InnovOx TOC Analyzer. Samples were acidified with phosphoric acid (6M). Sodium persulfate solution (30 wt %) was used as the oxidizer. Potassium hydrogen phthalate and mannitol were used for calibration of the instrument.

Various terms and designations used in the following examples are explained herein as follows: "DVB" stands for divinylbenzene; "DVBDO" stands for divinylbenzene dioxide; "DVBMO" stands for divinylbenzene monooxide; and "EVB" stands for ethylvinylbenzene. The stir rate is measured in revolutions per minute, abbreviated as rpm.

For each of the following preparations in the Examples, 80 % DVB was used containing 20 % EVB, but the yields and final compositions were referred to DVB.

### Synthesis Example 1

Caro's acid was prepared based on the procedure described in Owusu, Hydrometallurgy 1998 48 91-99. Caro's acid is partially neutralized with a potassium hydroxide as follows:
Caro's acid solution was kept in a 500 mL beaker. A 40 % aqueous potassium hydroxide solution (52 g) was added to the solution over 1 hour at 0 °C. The solution was stirred for an additional 1 hour with the temperature being held between 0 °C and 5 °C. The solution was allowed to warm up to room temperature (about 25 °C) and stirred for an additional 1 hour. The resulting reaction mixture may be directly used in a subsequent epoxidation step. Alternatively, the solution can be transferred to a container and stored in a refrigerator until further use.

### Synthesis Example 2

Caro's acid was prepared based on the procedure described in Owusu, Hydrometallurgy 1998 48 91-99. To the Caro's acid solution, kept in a 500 mL beaker, a sodium hydroxide solution (10 wt %, 160 g) was added over 1 hour at 0 °C. The solution was allowed to warm up to room temperature (about 25 °C) and stirred for an additional 1 hour. The resulting reaction mixture may be directly used in a subsequent epoxidation step; or alternatively, the resulting solution can be transferred to a plastic container and stored in a refrigerator.

### Example 1

In a 250 mL 3 neck flask equipped with a mechanical stirrer, pH meter, and thermocouple, divinylbenzene (1.03 g, 7.9 mmol), acetone (82 mL), and water (42 mL) were added and stirring commenced at ambient temperature. To the flask, sodium bicarbonate (5.50 g, 63 mmol) in water (30 mL) was added followed by an additional mixing time of 10 minutes. Then partially neutralized Caro's acid (3.35 g, 20.2 mmol) from Synthesis Example 1 was added to the reaction mixture in portions over 15 minutes. The pH of the reaction mixture was maintained at between 7 and 8. After 2 hours, divinylbenzene was fully consumed. There was no DVDMO detected in the reaction mixture by gas chromatography and the reaction mixture contained 96 % DVBDO, disregarding EVBO components.

### Example 2

DVB ( 1.13 g, 7.92 mmol), acetone (81 mL), water (42 mL), and sodium bicarbonate (5.42 g, 63.2 mmol) were transferred to a flask equipped with a mechanical stirrer (300 rpm), a thermocouple and pH meter. Partially neutralized Caro's acid from Synthesis Example 2 was added to the reaction mixture over the course of 30 minutes. Synthesis Example 2 or sodium peroxymonsulfate (2.94 g, 19.75 mmol) was added to the reaction. The reaction mixture was kept at 25 °C. The pH of the solution was kept between 7 and 8. The reaction mixture was analyzed by GC. After 3 hours divinylbenzene was fully consumed. There was no DVDMO detected in the reaction mixture and the reaction mixture contained 95 % DVBDO, disregarding EVBMO components.

### Example 3

The epoxidation procedure of Example 2 was repeated, except 2.5 fold molar excess of ActXone™ and 3 fold molar excess of sodium hydrogen carbonate were used per mol of DVB. The reaction was completed in five hours and 98 % DVBDO was obtained.

### Example 4

The epoxidation procedure of Example 3 was repeated, except eight fold molar excess of sodium hydrogen carbonate was used per mol of DVB. The reaction was completed in one hour and 98 % DVBDO was obtained.

### Example 5

DVB (1.03 g, 7.9 mmol), acetone (75 mL) and a solution of dibasic sodium phosphate (0.25M, 60 mL) were transferred to a flask equipped with a mechanical stirrer (300 rpm), thermocouple and pH meter. Partially neutralized Caro's acid (ActXone) (23.7 mmol, 30 % solution, 10.53 g) was delivered into the reaction mixture in the course of 20 minutes maintaining the temperature at 25 °C. The pH of the solution was continuously monitored and kept at 7-8 using potassium hydroxide (1.5 M). The reaction mixture was further incubated and analyzed by GC. After 1 hour, DVB was completely converted to products, no DVB and DVBMO were present and the reaction mixture contained 100 % DVBDO, disregarding EVBMO related components.

DVBDO was isolated by filtering the solids and extracting the acetone-water reaction mixture with methylene chloride (250 mL). The resulting methylene chloride solution was then washed with sodium bicarbonate (250 mL, 8 % solution), then the methylene chloride solution was washed with water (250 mL); and then the resulting methylene chloride solution was dried over sodium sulfate. The solvents were removed with rotary evaporation. DVBDO product yield was 95 %. The mixture was vacuum distilled to separate the DVBDO product from EVBMO. Vacuum distillation resulted in a 10 % DVBDO loss; and 98 % pure DVBDO.

### Example 6

DVB (0.78 g, 6.0 mmol), acetone (44 mL) and sodium hydrogen carbonate (3.07 g, 36 mmol) in water (38 mL) were transferred to a flask equipped with a mechanical stirrer (300 rpm), thermocouple and pH meter. Partially neutralized Caro's acid (ActXone) (15.0 mmol, 30 % solution, 6.67 g) was delivered into the reaction mixture in the course of 20 minutes maintaining the temperature at 25 °C. The pH of the solution was continuously monitored and kept at 7-8. The reaction mixture was further incubated and analyzed by GC. After 1 hour, DVB was completely converted to products, no DVB and DVBMO present and the reaction mixture contained 95 % DVBDO, disregarding EVBMO components.

### Example 7

In this example 7 DVB is epoxidized with partially neutralized Caro's acid as follows: DVB (6.50 g, 50.0 mmol), acetone (36.3 g) and potassium hydrogen carbonate (17.5 g, 175.0 mmol) and water (36 mL) were transferred to a flask equipped with a mechanical stirrer (600 rpm), thermocouple and pH meter. Partially neutralized Caro's acid or potassium peroxomonosulfate (125.0 mmol, 60.70 g) was delivered into the reaction mixture in the course of 60 minutes using a peristaltic pump and maintaining the temperature at 25 °C. The pH of the solution was continuously monitored and was between 7-8. After oxidant delivery, the reaction mixture was further incubated for an hour. Salt, precipitated during the reaction was filtered using a Buchner vacuum funnel system and Whatman #1 filter paper. It was washed with toluene (25 g) three times. After filtration the salt was dried in a vacuum over resulting in 26.99 g dry salt. The aqueous acetone filtrate was extracted with toluene. The toluene extract and toluene used for the salt wash were mixed and washed with water (100 mL) and dried over sodium sulfate. Toluene was evaporated using a rotary evaporator yielding DVBDO as a pale yellow liquid (6.84 g, 87 %), disregarding EVBMO components.

### Example 8

In this example 8 DVB is epoxidized with partially neutralized Caro's acid as follows: DVB (65.00 g, 0.5 mol), acetone (363 mL, 6.3 mol), NaHCO₃ (147.01 g, 1.8 mol) and water (360.06 mL) were transferred to a flask with indentations, equipped with a mechanical stirrer, thermocouple, addition port and condenser. A solution of KHSO₅ (607.3 g, 1.3 mol) was pumped into the reaction mixture in the course of 1 hour maintaining the temperature at 25 °C and stirring at 600 rpm. The reaction mixture was further incubated for an additional hour. After 2 hours stirring was stopped and the pH of the solution was checked and was 7.7. Salts precipitated were filtered using a Buchner funnel equipped with Watman #1 paper. Salt was washed with acetone (3 times 200 g). The salt was dried under vacuum yielding 296 g dry salt with a TOC of 518 ppm.

The filtrate was extracted with toluene (2x 250 g). The unified organic solutions (extract and salt wash) were washed with water ( 227 mL). The organic layer was dried over sodium sulfate. The solvent was removed with rotary evaporation, yielding pale yellow DVBDO product (73.0 g, 92 %, disregarding EVBMO components). The product was also analyzed by GC and GPC. DVB was completely converted to products and no DVBMO was present. DVBDO was 96 % pure by GC and contained 4 % oligomers by gel permeation chromatography.

### Example 9

The epoxidation procedure of Example 8 was repeated, except the KHSO₅ solution was pumped into the reaction mixture in the course of 2 hours maintaining the temperature at 25 °C and stirring at 600 rpm. The reaction mixture was further incubated for an additional hour, by then the reaction was completed indicated by gas chromatography showing 98 % DVBDO yield, disregarding EVBMO components.

### Example 10

In this example 10 DVB is epoxidized with partially neutralized Caro's acid as follows: DVB (3.01 g, 23.0 mmol), acetone (30.02 g, 517 mmol), NaHCO₃ (8.72 g, 103.7 mmol) and water (96.80 mL) were transferred to a flask, equipped with a mechanical stirrer, pH meter, thermocouple, addition port and condenser. A solution of KHSO₅ (15.43 g, 33.5 mmol) was pumped into the reaction mixture in the course of 10 minutes. The reaction pH was 7.4. The reaction mixture was stirred at 300 rpm and 25 °C temperature. After an hour reaction time, the pH reached 8.4. The reaction mixture was sampled and the residual amount of KHSO₅ was determined iodometrically. There was none detected. Additional amount of KHSO₅ solution was added (4.09 g, 8.9 mmol) until the pH decreased to 7.4. The reaction mixture was further incubated at 25 °C for two additional hours when the pH again raised to 8.6. The reaction mixture was again sampled and the residual amount of KHSO₅ was determined iodometrically. There was none detected. Additional amount of KHSO₅ solution was added (2.98 g, 6.5 mmol) until the pH decreased to 7.3. The reaction mixture was further incubated at 25 °C for an additional hour when the pH again raised to 8.3. GC analyses still showed the presence of DVBMO. Iodometric titration of a sample of the reaction mixture did not show any residual oxidant present, so an additional amount of KHSO₅ solution (3.00 g 6.5 mmol) was delivered that decreased the pH to 7.1. After an additional hour of incubation no more DVBMO was observed by gas chromatography and 97 % DVBDO was produced disregarding EVBMO.

### Example 11

The epoxidation procedure of Example 2 was repeated, except 2.5 fold molar excess of ActXone™ and 3.4 fold molar excess of sodium hydrogen carbonate were used per mol of DVB.

DVBDO was isolated in the following manner. After the reaction was completed as determined by GC, the salts were filtered via vacuum filtration via Buchner funnel. Acetone (10 mL) was added to rinse out the residual salt from the round bottom flask. The filtrate was transferred to a round bottom flask. The organic volatiles were removed via rotary evaporation. The organic solution was phase separated from the water component to isolate DVBDO whose purity and yield were checked by GC. DVBDO was obtained as a pale-yellow liquid. DVBDO was produced in 97 % purity disregarding EVBMO.

### Example 12

Example 8 was repeated except that instead of using NaHCO₃, KHCO₃ was used as the buffer. The sulfate by-product was filtered, divided into three portions and purified in three different ways.

### Purification 1

The salt was washed with toluene and the TOC of the salt was determined after each washing cycle. After the first wash the TOC was 494 ppm, which decreased to 127 ppm after the second wash and was measured to be 70 ppm after the third wash.

### Purification 2

The salt was dissolved in water and the solution was extracted with dichloromethane. Than the water was evaporated and the TOC of the residual salt was measured as 14 ppm.

### Purification 3

The salt was calcined at 300 °C for two hours. The TOC was 39 ppm.

## Claims

1. A process for preparing a divinylarene dioxide comprising reacting:
(a) a divinylarene;
(b) a partially neutralized sulfuromonoperoxoic acid as an oxidant;
(c) a basic compound;
(d) a ketone which reacts with the partially neutralized sulfuromonoperoxoic acid to form a dioxirane oxidant and which solubilizes the organic compounds present in the reaction mixture;
wherein the ketone is selected from acetone; methyl-ethyl ketone; acetophenone; trifluoroacetone; trifluoro acetophenone; tetrafluoro acetophenone; or mixtures thereof; wherein the dioxirane oxidant is isolated and then the dioxirane oxidant is used in a subsequent process; or wherein the dioxirane is used *in situ* as the oxidant in the presence of the divinylarene mixed with the ketone during the addition of partially neutralized sulfuromonoperoxoic acid; and
wherein the process is carried out under conditions to form a divinylarene dioxide product.

2. The process of claim 1, wherein the divinylarene is divinylbenzene; and wherein the divinylarene dioxide formed is divinylbenzene dioxide.

3. The process of claim 1, wherein the partially neutralized sulfuromonoperoxoic acid is obtained by neutralizing sulfuromonoperoxoic acid with (i) inorganic base, (ii) ammonium hydroxide, (iii) alkylammonium base; (iv) arylammonium base; (v) mixed alkyl-arylammonium base; (vi) alkylphosphonium base; (vii) arylphosphonium base; (viii) mixed alkyl-aryl phosphonium base; or (ix) mixtures thereof.

4. The process of claim 3, wherein the sulfuromonoperoxoic acid is neutralized with potassium hydroxide, sodium hydroxide, ammonium hydroxide, potassium carbonate, sodium carbonate, ammonium carbonate, tetrabutylammonium hydroxide, tetraphenylammonnium hydroxide, or mixtures thereof.

5. The process of claim 1, where the basic compound comprises an inorganic base or an inorganic salt; and wherein the basic compound comprises sodium hydrogencarbonate; potassium hydrogencarbonate; sodium hydroxide; potassium hydroxide; sodium carbonate; potassium carbonate; sodium phosphate; potassium phosphate; sodium borate; potassium borate; or mixtures thereof.

6. The process of claim 1, wherein the ketone is acetone.

7. The process of claim 1, wherein the yield of divinylarene dioxide is at least 60 percent; and wherein the minor product in the reaction is a divinylarene monooxide comprising a yield of less than 40 percent.

8. The process of claim 1, including the steps of: separating the divinylarene dioxide product formed from the reaction mixture; and purifying the divinylarene dioxide product obtained.

9. The process of claim 1, including adding one or more free radical polymerization inhibitors to the reaction mixture.

10. The process of claim 9, wherein the product purifying step is carried out by distillation.

11. The process of claim 1, including the steps of recovering and purifying a by-product salt formed in the reaction mixture.

12. The process of claim 11, wherein the step of purifying the salt by-product is carried out by calcination; crystallization; organic solvent extraction; adsorption of the impurities on activated carbon, or a combination thereof.

13. The process of claim 1, including the steps of: recovering and/or recycling the ketone from the reaction mixture.

## Patentansprüche

1. Ein Verfahren zum Herstellen eines Divinylarendioxids, beinhaltend das zur Reaktion bringen:
(a) eines Divinylarens;
(b) einer teilweise neutralisierten Peroxomonoschwefelsäure als ein Oxidationsmittel;
(c) einer basischen Verbindung;
(d) eines Ketons, welches mit der teilweise neutralisierten Peroxomonoschwefelsäure unter Bildung eines Dioxiran-Oxidationsmittels reagiert, und welches die in der Reaktionsmischung vorliegenden organischen Verbindungen löst;
wobei das Keton ausgewählt wird aus Aceton; Methylethylketon; Acetophenon; Trifluoraceton; Trifluoracetophenon; Tetrafluoracetophenon, oder Mischungen davon; wobei das Dioxiran-Oxidationsmittel isoliert wird und das Dioxiran-Oxidationsmittel danach in einem nachfolgenden Verfahren verwendet wird; oder wobei das Dioxiran *in situ* als das Oxidationsmittel in Gegenwart des mit dem Keton vermischten Divinylarens während des Hinzufügens von teilweise neutralisierter Peroxomonoschwefelsäure verwendet wird; und
wobei das Verfahren unter Bedingungen zum Bilden eines Divinylarendioxid-Produkts durchgeführt wird.

2. Verfahren gemäß Anspruch 1, wobei das Divinylaren Divinylbenzol ist; und wobei das gebildete Divinylarendioxid Divinylbenzoldioxid ist.

3. Verfahren gemäß Anspruch 1, wobei die teilweise neutralisierte Peroxomonoschwefelsäure durch Neutralisieren von Peroxomonoschwefelsäure mit (i) anorganischer Base, (ii) Ammoniumhydroxid, (iii) Alkylammoniumbase; (iv) Arylammoniumbase; (v) gemischter Alkylarylammoniumbase; (vi) Alkylphosphoniumbase; (vii) Arylphosphoniumbase; (viii) gemischter Alkylarylammoniumbase; oder (ix) Mischungen davon erhalten wird.

4. Verfahren gemäß Anspruch 3, wobei die Peroxomonoschwefelsäure mit Kaliumhydroxid, Natriumhydroxid, Ammoniumhydroxid, Kaliumcarbonat, Natriumcarbonat, Ammoniumcarbonat, Tetrabutylammoniumhydroxid, Tetraphenylammoniumhydroxid, oder Mischungen davon neutralisiert wird.

5. Verfahren gemäß Anspruch 1, wobei die basische Verbindung eine anorganische Base oder ein anorganisches Salz beinhaltet; und wobei die basische Verbindung Natriumhydrogencarbonat, Kaliumhydrogencarbonat, Natriumhydroxid, Kaliumhydroxid, Natriumcarbonat, Kaliumcarbonat, Natriumphosphat, Kaliumphosphat, Natriumborat, Kaliumborat; oder Mischungen davon beinhaltet.

6. Verfahren gemäß Anspruch 1, wobei das Keton Aceton ist.

7. Verfahren gemäß Anspruch 1, wobei die Ausbeute an Divinylarendioxid mindestens 60 Prozent beträgt; und wobei das Nebenprodukt in der Reaktion ein Divinylarenmonoxid ist, eine Ausbeute von weniger als 40 Prozent beinhaltend.

8. Verfahren gemäß Anspruch 1, einschließlich der Schritte: Trennen des gebildeten Divinylarendioxid-Produkts von der Reaktionsmischung; und Reinigen des erhaltenen Divinylarendioxid-Produkts.

9. Verfahren gemäß Anspruch 1, einschließlich Zufügen eines oder mehrerer Radikalpolymerisationsinhibitoren zur Reaktionsmischung.

10. Verfahren gemäß Anspruch 9, wobei der Produktreinigungsschritt mittels Destillation durchgeführt wird.

11. Verfahren gemäß Anspruch 1, einschließlich der Schritte des Rückgewinnens und Reinigens eines in der Reaktionsmischung gebildeten Nebenproduktsalzes.

12. Verfahren gemäß Anspruch 11, wobei der Schritt des Reinigens des Nebenproduktsalzes durch Kalzination; Kristallisation; Extraktion mit organischem Lösemittel; Adsorption der Verunreinigungen an Aktivkohle, oder einer Kombination davon durchgeführt wird.

13. Verfahren gemäß Anspruch 1, einschließlich der Schritte des: Rückgewinnens und/oder Wiederverwertens des Ketons von der Reaktionsmischung.

## Revendications

1. Un procédé pour préparer un dioxyde de divinylarène comprenant la mise en réaction :
(a) d'un divinylarène ;
(b) d'un acide peroxymonosulfurique en partie neutralisé en tant qu'oxydant ;
(c) d'un composé basique ;
(d) d'une cétone qui réagit avec l'acide peroxymonosulfurique en partie neutralisé afin de former un oxydant dioxirane et qui solubilise les composés organiques présents dans le mélange réactionnel ;
dans lequel la cétone est sélectionnée parmi l'acétone ; le méthyléthylcétone ; l'acétophénone ; le trifluoroacétone ; le trifluoroacétophénone ; le tétrafluoroacétophénone ; ou des mélanges de ceux-ci ;
dans lequel l'oxydant dioxirane est isolé et ensuite l'oxydant dioxirane est utilisé dans un procédé ultérieur ; ou dans lequel le dioxirane est utilisé in situ en tant qu'oxydant en présence du divinylarène mélangé avec la cétone au cours de l'ajout d'acide peroxymonosulfurique en partie neutralisé ; et
dans lequel le procédé est réalisé dans des conditions aptes à former un produit dioxyde de divinylarène.

2. Le procédé de la revendication 1, dans lequel le divinylarène est le divinylbenzène ; et dans lequel le dioxyde de divinylarène formé est le dioxyde de divinylbenzène.

3. Le procédé de la revendication 1, dans lequel l'acide peroxymonosulfurique en partie neutralisé est obtenu en neutralisant de l'acide peroxymonosulfurique avec (i) une base inorganique, (ii) de l'hydroxyde d'ammonium, (iii) une base alkylammonium ; (iv) une base arylammonium ; (v) une base alkyl-arylammonium mélangée ; (vi) une base alkylphosphonium ; (vii) une base arylphosphonium ; (viii) une base alkyl-arylphosphonium mélangée ; ou (ix) des mélanges de ceux-ci.

4. Le procédé de la revendication 3, dans lequel l'acide peroxymonosulfurique est neutralisé avec de l'hydroxyde de potassium, de l'hydroxyde de sodium, de l'hydroxyde d'ammonium, du carbonate de potassium, du carbonate de sodium, du carbonate d'ammonium, de l'hydroxyde de tétrabutylammonium, de l'hydroxyde de tétraphénylammonium, ou des mélanges de ceux-ci.

5. Le procédé de la revendication 1, dans lequel le composé basique comprend une base inorganique ou un sel inorganique ; et dans lequel le composé basique comprend de l'hydrogénocarbonate de sodium ; de l'hydrogénocarbonate de potassium ; de l'hydroxyde de sodium ; de l'hydroxyde de potassium ; du carbonate de sodium ; du carbonate de potassium ; du phosphate de sodium ; du phosphate de potassium ; du borate de sodium ; du borate de potassium ; ou des mélanges de ceux-ci.

6. Le procédé de la revendication 1, dans lequel la cétone est de l'acétone.

7. Le procédé de la revendication 1, dans lequel le rendement du dioxyde de divinylarène est d'au moins 60 pour cent ; et dans lequel le produit minoritaire dans la réaction est un monoxyde de divinylarène comprenant un rendement inférieur à 40 pour cent.

8. Le procédé de la revendication 1, incluant les étapes consistant à : séparer le produit dioxyde de divinylarène formé du mélange réactionnel ; et purifier le produit dioxyde de divinylarène obtenu.

9. Le procédé de la revendication 1, incluant l'ajout d'un ou de plusieurs inhibiteurs de la polymérisation radicalaire au mélange réactionnel.

10. Le procédé de la revendication 9, dans lequel l'étape de purification de produit est effectuée par distillation.

11. Le procédé de la revendication 1, incluant les étapes consistant à récupérer et purifier un sel sous-produit formé dans le mélange réactionnel.

12. Le procédé de la revendication 11, dans lequel l'étape de purification du sel sous-produit est effectuée par calcination ; cristallisation ; extraction de solvant organique ; adsorption des impuretés sur du charbon actif, ou une combinaison de celles-ci.

13. Le procédé de la revendication 1, incluant les étapes consistant à : récupérer et/ou recycler la cétone du mélange réactionnel.
